# EUROPEAN PATENT SPECIFICATION

(11) **EP 1 226 436 B1**
(45) Date of publication and mention of the grant of the patent: **20.04.2005**
(21) Application number: 00973067.2
(22) Date of filing: 06.11.2000
(51) Int. Cl.: G01N 33/542, G01N 33/533, G01N 33/58, C12N 15/00

(54) **UNIVERSAL FLUORESCENT SENSORS**
UNIVERSELLE FLUORESZENZSENSOREN
MARQUEURS FLUORESCENTS UNIVERSELS

(30) Priority: 05.11.1999 GB 9926336
(43) Date of publication of application: 31.07.2002
(73) Proprietor: ISIS INNOVATION LIMITED, Summertown, Oxford OX2 7SG (GB)
(72) Inventor: FRICKER, Mark David, University of Oxford, Oxford OX1 3RB (GB); VAUX, David John Talbutt, University of Oxford, Oxford OX1 3RB (GB)
(74) Representative: Ellis-Jones, Patrick George Armine
(86) International application number: PCT/GB2000/004255
(87) International publication number: WO 2001/033199

(56) References cited:
- EP-A- 0 552 108
- WO-A-89/09408
- WO-A-96/29367
- WO-A-98/40477
- WO-A-98/48278
- US-A- 5 246 867

## Description

The invention relates to probes which are used for the detection of a wide range of substances. The invention also relates to probes which are used for the identification of inhibitors which reduce binding between two substances which bind to each other in the absence of an inhibitor.

Probes of the invention can be used in, for example, medical diagnosis, the detection of pollutants in water systems and the detection of contaminants in foodstuffs and in animal and plant biology. They can also be used in the identification of new therapeutic substances.

### Background to The Invention

When a fluorescent molecule absorbs light, an electron is excited to a higher energy level. Typically the electron loses some energy before decaying back to ground state. During this transition, a photon is emitted with less energy than the excitation photon and hence at a longer wavelength. If a second fluorophor is in close proximity, the energy released by the electron as it decays in the donor fluorophor may be transferred directly to the second acceptor fluorophor and excite one of the electrons of the latter to a higher energy level. When the electron in the acceptor decays from this state, an even longer wavelength photon is released. The process is termed fluorescence resonance energy transfer (FRET). The extent to which FRET takes place is critically dependent on the overlap of the spectra between the two fluorophors and their separation. Thus, FRET decreases roughly in proportion to the sixth power of the separation between the two fluorophors and is a powerful reporter for the separation of the two fluorophors at the molecular level.

The coding sequence for a range of fluorescent proteins is now available and some of these proteins have an appropriate overlap in then emission and excitation spectra for efficient FRET to take place. Heim and Tsien (1996, Curr. Biol. **6**, 178-182), have demonstrated that FRET can occur between two such fluorescent proteins when they are tethered together and the FRET signal alters if the peptide linker is severed by a protease. Using this principle Miyawaki *et al*. (1997, Nature **388**, 882-887) demonstrated the use of a FRET-based indicator for calcium detection. This was achieved by using the calcium-binding protein (calmodulin) and a short calmodulin-binding target sequence (M13) as part of the linker between the two fluorophors. Calmodulin undergoes a conformational change on binding calcium and subsequently binds to the adjacent calmodulin-binding sequence. This serves to after the separation of the fluorescent proteins and modulates the level of FRET.

Although the potential exists to generate probes for other molecules, identification and screening of proteins or protein motifs with appropriate properties to both bind to the target and to alter the separation of the fluorophors is not straightforward.

### Summary of The Invention

According to the invention there is provided a probe comprising:
(i) a target binding site peptide which is attached to a first fluorescent polypeptide;
(ii) a mimic peptide which is capable of binding to the target binding site peptide and is attached to a second fluorescent polypeptide; and
(iii) a linker which connects the two fluorescent polypeptides and which allows the distance between said fluorescent polypeptides to vary, said fluorescent polypeptides being such as to display fluorescence resonance energy transfer (FRET) between them.

The invention also provides:
- a polynucleotide which encodes a probe of the invention;
- a vector incorporating a polynucleotide of the invention;
- a cell harbouring a probe, polynucleotide or vector of the invention;
- a fungus, plant or non-human animal comprising a probe, polynucleotide, vector or cell of the invention;
- a sensor comprising:
   (i) a probe of the invention;
   (ii) a light source which is capable of exciting the probe;
   (iii) a detector which is capable of measuring the amount of FRET from the probe;
- a method for detecting the presence or absence of a target substance in a test sample comprising:
   (i) providing a probe, cell or sensor of the invention, wherein the target binding site peptide of the probe, cell or sensor is capable of binding to the target substance;
   (ii) determining the amount of FRET of the probe, cell or sensor;
   (iii) contacting the probe, cell or sensor with the test sample; and
   (iv) determining any change in FRET thereby to determine whether the test sample comprises the target substance;
- use of a probe, cell or sensor of the invention, wherein the target binding site peptide of the probe, cell or sensor is capable of binding to a target substance, in the detection of the presence or absence of that target substance in a test sample;
- a method for identifying an inhibitor of binding between two substances, which two substances would bind to each other in the absence of an inhibitor, composing
   (i) providing a probe or cell of the invention, wherein the binding of the target site peptide to the mimic peptide mimics the binding of the two substances to each other;
   (ii) determining the amount of FRET of the probe or cell;
   (iii) contacting the probe or cell with a test substance; and
   (iv) determining any change in FRET thereby to determine whether the test substance inhibits binding between the two substances; and
- use of a probe or cell according to the invention, wherein the binding of the target site peptide to the mimic peptide mimics the binding of two substances to each other, in the identification of an inhibitor of binding between those two substances.

### Brief Description of The Figures

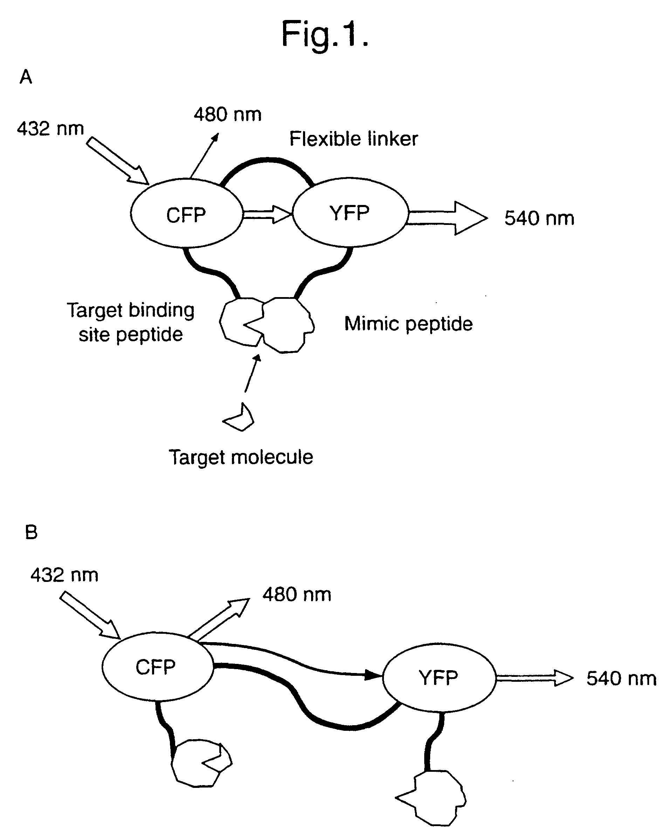

Figure 1 shows the design and principle of operation of the probes.
   A. In the absence of the target compound, the mimic peptide binds to the target binding site peptide. The linker allows the two fluorophors to approach each other and a high level of FRET results.
   B The target molecule competes with the mimic for the target binding site causing separation of the two fluorophors and a decrease in FRET.

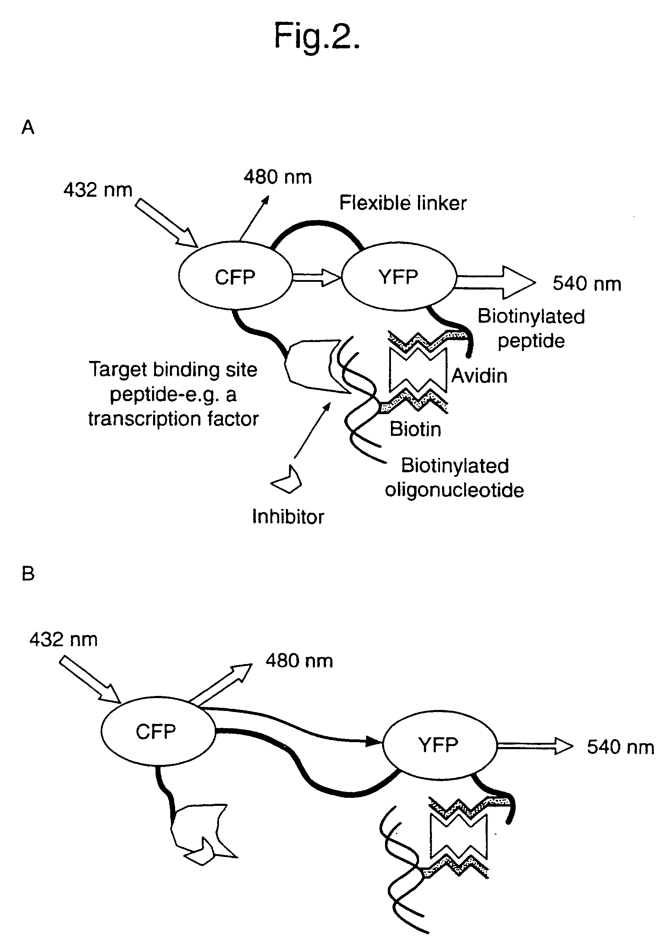

Figure 2 shows the design of biotinylated probes.
   A. The mimic peptide comprises a sequence capable of biotinylation. Avidin binds to the biotinylated probe and the resulting complex can subsequently bind with a biotinylated substrate. A biotinylated oligonucleotide is shown bound to the target peptide, which is, for example, a transcription factor.
   B. An inhibitor binds to the transcription factor, disrupting the binding between the oligonucleotide and the transcription factor. The distance between the two fluorescent peptides increases and FRET is thus reduced.

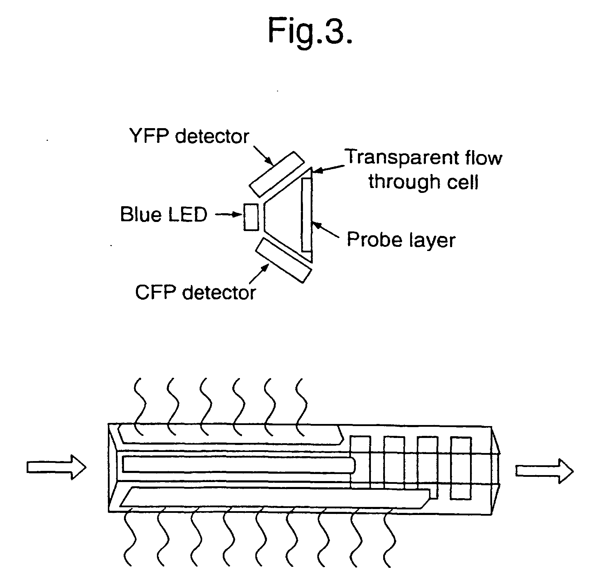

Figure 3 shows the arrangement of the excitation source (a blue LED, blue laser or other appropriate light source) and two detectors around a flow-through cell containing pads of sensors to different compounds in a flow-through array detector which may be used in the present invention.
   A. Cross section.
   B. Surface view.

### Detailed Description of The Invention

The probes of the invention comprise two fluorescent polypeptides connected by a linker. A target binding site peptide is attached to one of the fluorescent polypeptides. A mimic peptide is attached to the other fluorescent polypeptide. The mimic peptide has a three dimensional structure that is complementary to the structure of target binding site peptide. Thus the mimic peptide can bind to the target binding site peptide.

The arrangement of the various domains of the probes is such that, typically, the target binding site and mimic peptides are free to interact with each other. When the target binding site and mimic peptides bind, the separation of the two fluorescent polypeptides (the fluorophors) is reduced and FRET occurs.

In the presence of a substance that disrupts the binding of the target site peptide to the mimic peptide, the separation between the target binding site peptide and mimic peptide may increase and consequently the separation between the fluorophors may also increase. As the separation of fluorophors increases, the level of FRET is reduced.

Probes of the invention may be designed so as to detect substantially any substance. In such probes the target binding site is capable of binding the substance, ie, the "target substance", which the probe is designed to detect. The mimic peptide binds to the target binding site peptide in a way that mimics the binding of the target substance to the target binding site peptide.

In the absence of the target substance, the target binding site and mimic peptides are free to bind with each other, the separation of the fluorescent polypeptides is reduced and FRET occurs (Figure 1A). In the presence of the target substance, that substance will compete with the mimic peptide for binding with the target binding site peptide and displace the mimic peptide from the target binding site. If the target substance displaces the mimic peptide, the separation between the target binding site peptide and the mimic peptide increases, the separation of the fluorophors increases and the amount of FRET is thus reduced (Figure 1B).

The degree or mimic peptide displacement increases as the amount of target substance increases and thus probes of the invention may provide a quantitative, as well as qualitative indication of the amount of target substance.

Probes of the invention may also be designed to screen for inhibitors which are capable of disrupting, reducing or even preventing two substances from binding to each other, which two substances in the absence of an inhibitor would bind to each other. In such probes the target binding site peptide and mimic peptide are chosen such that the way in which they interact mimics the binding interaction of the two substances of interest.

In the absence of the inhibitor, the target binding site and mimic peptide of an appropriate probe are free to bind with each other. The separation of the fluorophors is reduced and FRET occurs. In the presence of an inhibitor the binding of the target binding site peptide and the mimic peptide is disrupted. The separation of the target binding site peptide and the mimic peptide increases and FRET is reduced.

Combinatorial libraries of chemicals, for example, may be screened to identify inhibitors within those libraries that can disrupt the binding of substantially any two substances that in the absence of an inhibitor will bind to each other.

Probes of the invention may also be designed to screen for stimulators, which increase or promote binding, between two substances. In such probes, the target binding site peptide and mimic peptide are chosen such that they mimic the two substances of interest.

In the absence of the stimulator, target binding site peptide and mimic peptide of an appropriate probe may bind to each other weakly or not at all. Thus, the separation of the fluorophors may be such that there is no FRET or FRET levels are low. In the presence of a stimulator, the target binding site and mimic peptides bind to each other or bind to each other more strongly than in the absence of the stimulator. The separation between the fluorophors is reduced and FRET is increased.

Thus, probes of the invention may be used to identify, for example, a factor which increases the strength of binding between two substances or, a factor whose presence is necessary for the binding of two substances to take place. Combinatorial libraries, for example, may be screened to identify stimulators of binding interactions.

In summary, a probe of the invention comprises five domains: a domain that binds the target substance (the target binding site peptide), a domain that binds to the target site (the mimic peptide), a donor fluorescent polypeptide, a linker and an acceptor fluorescent polypeptide.

Typically the linker is a peptide/polypeptide and is connected to the two fluorescent polypeptides by peptide bonds. Also the target binding site peptide and mimic peptide are typically attached to their respective fluorescent polypeptides by peptide bonds. Thus a probe of the invention is typically a single polypeptide. When a probe is a single polypeptide, polynucleotides may be obtained which encode the probes. Such polynucleotides can be used in the manufacture of probes by, for example, expression in bacteria or transcription and translation of the polynucleotides in cell-free systems.

The mimic peptide can be connected to a non-protein substance, or may comprise a peptide sequence which is capable of being connected to a non-protein substance. For example, the mimic peptide can comprise a sequence capable of biotinylation. A probe comprising a biotinylation target sequence can be biotinylated and subsequently bound to streptavidin. Addition of a biotinylated substrate to a probe-streptavidin complex gives rise to the formation of a probe-substrate complex. Typically, the target binding site peptide is capable of binding to the substrate and therefore such a probe may be used in detection of the substrate.

Such biotinylated probes provide a relatively straightforward route to the production of probes which can be used to detect the presence or absence of non-peptide components, including mRNA, DNA, carbohydrates, lipids and other organic molecules in test samples. Additionally, such probes may also be used to identify inhibitors of binding between two substances. Figure 2 shows how a biotinylated probe may be used to screen for an inhibitor of the binding interaction between a transcription factor and the nucleotide motif to which that transcription factor binds.

Biotinylated probes may produced by using a 17 residue biotin acceptor sequence that acts as a substrate for biotin ligase and permits the creation of endogenously biotinylated proteins. A suitable biotin acceptor sequence is MSGLNDIFEAQKIEWHE, which is based on the minimal acceptor sequence (Schatz, 1998, Biotechnology **11**, 1138-1143.) as adapted for higher affinity (Beckett *et al*., 1999, Protein Sci. **8**, 921-929). A polynucleotide construct encoding a probe, wherein the sequence encoding the mimic peptide comprises nucleotide sequence encoding the biotinylation sequence, can be expressed in a bacterial strain over-expressing BirA (biotin ligase). This results in the expression of a protein which is biotinylated. The protein can be biotinylated at the N- or C- terminal end, depending on the location of the biotinylation peptide. This technology has been developed by Avidity under the trade name Avitag (US-A-5,723,584).

Biotinylated probes can he purified on affinity columns comprising streptavidin bound to 2-imino-biotin attached to the column support. The probe-avidin complex is typically then released by dissociation from the 2-imino-biotin column support at low pH, for example pH4.0. The approach leads to a purified probe-streptavidin complex with a free binding site for biotin following release from the 2-imino-biotin column. Subsequent addition of any biotinylated substrate will allow reconstitution of complete probe.

Appropriate pairs of fluorescent polypeptides are those which exhibit FRET. That is, the donor polypeptide must be capable or absorbing light which excites an electron to a higher energy level. The electron will lose energy as it decays back to its ground state. The acceptor polypeptide must in turn be capable of accepting that energy to become excited itself. The extent to which FRET takes place is critically dependent on the overlap of the spectra of the fluorescent polypeptides and their separation. When selecting pairs of fluorescent polypeptides for use in a probe of the invention, various spectroscopic properties of the donor and acceptor need to be considered: (1) there needs to be sufficient separation in excitation spectra if the donor fluorescent polypeptide is to be stimulated selectively; (2) there needs to be an overlap between the emission spectrum of the donor and the excitation spectrum of the acceptor to obtain efficient energy transfer, and (3) reasonable separation in emission spectra between donor and acceptor fluorescent polypeptides is required to allow the fluorescence of each chromophore to be measured independently.

Suitable polypeptides include those from the green fluorescent protein (GFP) family of polypeptides, which are derived from the jellyfish species *Aequoria victoria*. Several basic classes of useful GFP mutants have been described, including: (1) red-shifted GFP, which has an emission peak most like that of wild-type GFP round 511nm, but lacks the near-UV 395nm excitation peak; (2) blue fluorescent protein (BFP); (3) cyan fluorescent protein (CFP), (4) sapphire; and (5) yellow fluorescent protein (YFP). For a review of GFPs see Pollok and Heim, 1999. TIBS 9, 57-60. Further GFP variants exist, for example a pH-insensitive CFP has been produced (Miyawaki *et al*. 1999, Proc. Natl. Acad. Sci. USA **96**, 2135-2140) The coding sequences for these polypeptides are known and those polynucleotide sequences may be used to produce the corresponding polypeptides. Further suitable fluorescent proteins may be used which are derived from species other than *Aequoria victoria*.

Suitable pairs of GFPs include BFP (as donor) and red-shifted GFP, CFP and YFP and pH-insensitive CFP and YFP. Further combinations of GFPs and of other types of fluorescent proteins may be derived empirically.

FRET can be measured by any method known to those in the art. For example, in the case of a probe comprising cyan fluorescent protein (CFP) and yellow fluorescent protein (YFP), excitation of CFP can be achieved by excitation with light at 430 to 440nm. Some of the energy may be transferred to the YFP by FRET. This energy is emitted at a much longer wavelength (540nm). Thus such a probe should be monitored at both 480nm and 540nm.

Any suitable light source may be used to cause excitation of the donor fluorescent polypeptide, for example a xenon are lamp, mercury are lamp, tungsten-halogen lamp, laser or LED. Light emission from both the donor and acceptor fluorescent polypeptides may be measured by any suitable detector, for example a photomultiplier, a silicon-detector, a charge-coupled device (CCD) detector, diode array or diode arrays or a CCD-camera or by surface plasmon resonance. Particular wavelengths may be selected using for example interference filters, absorption filters, dichroic mirrors, prisms or diffraction gratings. The light sources, detectors and wavelength selectors may be combined in currently available instruments including fluorimeters, fluorescent plate readers, photometry systems, confocal microscopes, multiphoton microscopes and ratio imaging devices.

The two fluorescent polypeptides are connected by a linker. Typically, the linker is sufficiently flexible to allow the separation between the fluorescent polypeptides to vary. Thus, altering the flexibility of the linker will typically alter the apparent binding affinity of the target and mimic. Therefore the nature of the linker will be an important determinant of the sensitivity of a probe of the invention.

Typically, the linker is a peptide/polypeptide. The flexibility of the linker will be influenced by the length of the linker and the precise amino acid composition of the linker. Suitable linker polypeptide sequences may comprise (Gly₃Ser)₄ and/or hinge sequences from heavy chains of antibodies.

Which of the fluorescent polypeptides (donor or acceptor) is attached to the target binding site peptide or the mimic peptide is generally immaterial. When the donor is attached to the target binding site peptide the acceptor is attached to the mimic peptide and *vice versa*. Typically, the target, binding site peptide and the mimic peptide are attached to their respective fluorescent polypeptides by peptide bonds. However, the target binding site peptide and the mimic peptide may be attached to the fluorescent polypeptides by other types of non-peptide bond connection.

Probes may comprise polypeptides which have been post-translationally modified. Thus, probes may comprise post-translational modifications such as phosphorylation, farnesylation or glycosylation. Probes may comprise more than one type of post-translational modification and may comprise up to 10, up to 20, up to 30, up to 50, up to 100 or more than 100 post-translational modifications.

Typically, a probe will comprise a single polypeptide and therefore a probe may be encoded by a single polynucleotide. The isolation of appropriate target-site and mimic peptides and the corresponding polynucleotides which encode those peptides allows the construction of polynucleotides encoding probes. Thus, the invention also provides polynucleotides which encode probes of the invention.

The invention further provides double stranded polynucleotides comprising a polynucleotide of the invention and its complement. Polynucleotides of the invention may comprise DNA or RNA. They may also be polynucleotides which include within them synthetic or modified nucleotides. A number of different types of modification to polynucleotides are known in the art. Such modifications may be carried out in order to enhance the in vivo activity, lifespan, nuclease resistance or ability to enter cells of polynucleotides of the invention. For example, phosphorothioate oligonucleotides may be used. Other deoxynucleotide analogs include methylphosphonates, phosphoramidates, phosphorodithioates, N3'P5'-phosphoramidates and oligoribonucleotide phosphorothioates and their 2'-O-alkyl analogs and 2'-O-methylribonucleotide methylphosphonates.

Alternatively mixed backbone oligonucleotides (MBOs) may be used. MBOs contain segments of phosphothioate oligodeoxynucleotides and appropriately placed segments of modified oligodeoxy- or oligoribonucleotides. MBOs have segments of phosphorothioate linkages and other segments of other modified oligonucleotides, such as methylphosphonate, which is non-ionic, and very resistant to nucleases or 2'-O-alkyloligoribonucleotides.

Polynucleotides such as a DNA polynucleotide according to the invention may be produced recombinantly, synthetically, or by any means available to those of skill in the art. They may also be cloned by standard techniques. The polynucleotides are typically provided in isolated and/or purified form. Although in general such techniques are well known in the art, reference may be made in particular to Sambrook *et al*., 1989. Molecular Cloning: A Laboratory Manual.

Polynucleotides of the invention can be incorporated into a recombinant replicable vector. The vector may be used to replicate the nucleic acid in a compatible host cell. Thus, in a further embodiment, the invention provides a method of making probes of the invention by introducing a polynucleotide of the invention into a replicable vector, introducing the vector into a compatible host cell, and growing the host cell under conditions which bring about replication of the vector. The vector may be recovered from the host cell.

Preferably, a polynucleotide of the invention in a vector is operably linked to control sequences which are capable of providing for the expression of that polynucleotide by the host cell, i.e. the vector is an expression vector. The term "operably linked" refers to a juxtaposition wherein the components described are in a relationship permitting them to function in their intended manner. Regulatory sequences, such as promoters and terminators, "operably linked" to a polynucleotide are positioned in such a way that expression of the polynucleotide is achieved under conditions compatible with the regulatory sequences. Typically regulatory sequences will comprise a promoter (generally positioned 5' to the polynucleotide), and/or a terminator and/or translation initiation sequence (eg. GCCACCATGG or GCCCCCATGG) and/or a translational stop codon (eg. TAA, TAG or TGA) and/or polyadenylation signal and/or one or more enhancer sequences and/or RNA pause site. The control sequences may increase transcription and or translation of the polynucleotide or may direct expression of the polynucleotide only in certain tissues.

The vectors may be, for example, plasmid, cosmid, virus or phage-vectors provided with an origin of replication, and optionally any of the control sequences described above. The vectors may contain one or more selectable marker genes, for example an ampicillin resistence gene may be used with a bacterial plasmid and a kanamycin resistance gene may be used with a plant vector.

Vectors may he used *in vitro*, for example for the production of RNA or introduced into a host cell. Any transfection or transformation technique may be performed in order to introduce a vector into a cell, for example, electroporation, salt precipitation, liposome mediated, protoplast fusion, viral infection, microinjection or ballistics techniques. The introduction may be aided by a natural mechanism by which the cell can take up material, such as pinocytosis or phagocytosis.

Thus, a further embodiment of the invention provides a host cell harbouring a vector of the invention. Cells transformed or transfected with vectors of the invention may allow for the replication and/or expression of polynucleotides encoding probes of the invention. Therefore, this invention also provides a cell harbouring a probe of the invention. The cell may be present in a culture of cells which culture also comprises a medium capable of supporting the cells.

The cells will be chosen to be compatible with the said vector and may be prokaryotic, such as a bacterial cell (eg. *E. coli*) or eukaryotic such as yeast, fungal, insect, plant, animal, for example, mammalian or human cells. The cell, may be undifferentiated or differentiated. The vector may exist in an episomal state in the host cell or the polynucleotide incorporated into the vector may become integrated into the genome of the cell.

Promoters and other control sequences may be selected to be compatible with the host cell for which expression is desired. For example, yeast promoters include *S. cerevisiae* GAL4 and ADH promoters, *S. pombe nmt*I and *adh* promoter. Plant promoters include the CAMV 35S and rubisco ssu promoters and mammalian promoters include the metallothionein promoter which can be induced in response to heavy metals such as cadmium. Viral promoters such as the SV40 large T antigen. promoter or adenovirus promoters may also be used for expression in mammals. All these promoters are readily available in the art.

The cell can be used in an expression system to produce the gene product. A preferred expression system is the baculovirus system. Thus, in a further aspect the invention provides a process for preparing a probe according to the invention, which comprises cultivating a host cell transformed or transfected with an expression vector incorporating a polynucleotide encoding the probe under conditions which allow expression of the probe, and optionally recovering the expressed probe

Probes of the invention can be designed to detect substantially any target substance. The target substance may be any substance for which an appropriate target binding site peptide-mimic peptide pair can be generated.

Probes may also be designed tu identify an inhibitor of binding between two substances, which two substances would bind to each other in the absence of an inhibitor. Substantially any binding interaction between two substances can be screened, providing that a target binding site peptide and mimic peptide pair can be generated, the binding to each other of which mimics the binding of the two substances of interest to each other.

Appropriate target binding site peptides may be isolated by any method and suitable methods will be well known to those skilled in the art. For example, antibodies specific for a particular target substance or specific to one of a pair of substances, whose binding to each other is to be investigated, may be isolated. This will allow the isolation of the coding sequences for appropriate single chain antibodies. Thus, it may be convenient to use antibodies from organisms that produce single chain antibodies, for example camels.

An alternative strategy for isolating target binding site peptides is to select sequences of proteins or protein motifs which have a defined substrate specificity. Also, sequences of proteins or protein motits that are glycosylated may provide suitable target binding site peptides for sugars and carbohydrates.

Similarly, mimic peptides may be isolated by any method and suitable methods will be well known to those skilled in the art. For example anti-idiotypic antibodies to the target substance may be generated and thus the coding sequences for appropriate single chain anti-idiotypic antibodies. Alternatively, mimic peptides may be generated by selecting sequences of proteins or protein motifs which have a defined substrate specificity.

A probe may be used to detect the presence or absence of a ligand by the use of an idiotype network. In this approach a pair of monoclonal antibodies is used, one of which is an internal image anti-idiotype of the other The method then requires the expression of each antibody as an ScFv, one as the target binding site peptide and the other as the mimic peptide. This gives rise to binding of the target binding site peptide to the mimic peptide and therefore a FRET signal in the resting state. The probe can then be used to specifically identify the original ligand (of which the anti-idiotype is an internal image) by a competition effect resulting in the loss of the FRET signal. This appproach is thus a general approach for the detection and measurement of any ligand with the specificity of the starting antibodies. It will be clear that the ligand does not necessarily have to be a peptide/protein. The ligand can be any substance for which the necessary pair of monoclonal antibodies can be generated.

Also, a combinatorial library of peptide sequences may be screened for binding to a target binding site peptide. Additionally a library of polynucleotides encoding probes could be produced, wherein the mimic peptide domain is encoded by a library of polynucleotides. The library can be screened for FRET Clones showing high levels of FRET should comprise polynucleotides which encode a mimic peptide which binds to the target-site peptide.

In the above techniques antibodies may be isolated by any suitable technique. For example, the target substance may be used to immunize an animal such as a rabbit, rat, mouse or chicken. Alternatively, expression libraries or phage display libraries may be screened. Those technique allow the convenient recovery of polynucleotides encoding suitable antibodies.

When a probe of the invention is used to detect the presence or absence of a particular target substance, the specificity of a probe will depend on the specificity of the target binding site peptide for the target substance and on the specificity of the mimic peptide. The sensitivity of the probe will depend on the dissociation constant of the binding site, the dissociation constant of the mimic-binding site interaction and the strain/flexibility imposed by the linker. Changing any one or more of these parameters should result in probes with a spread of specificities and sensitivities.

Typically, a probe will be specific for one target substance or a small number, for example 2. 3, 5 or 10 target substances. However, the invention also provides probes which are less specific and thus may be capable of detecting a family of substances, for example, at least 10, at least 20, or at least 50 substances. The family of substances will typically share similarity in an aspect of their structure.

Probes with high sensitivity are preferred. Changes in FRET are typically measured as changes in the ratio of donor fluorescence to acceptor fluorescence A reduction is FRET is typically indicated by an increase in the ratio of donor fluorescence to acceptor fluorescence. The greater the increase in the ratio of donor fluorescence to acceptor fluorescence on binding a target substance species, the greater the sensitivity of the probe. Preferred probes are those which, when one probe molecule binds one target substance species, exhibit a reduction in FRET and thus an increase in the ratio of donor fluorescence to acceptor fluorescence of from 1.5 to 2.0, preferably from 0.5 to 3.0 or more preferably from 0.1 to 5.0.

New probes can be screened to establish whether they exhibit FRET. Probes that exhibit FRET may be titrated with the target substances to determine the sensitivity. Probes may then be screened with substances related to the target substances to determine the probe specificity.

Probes of the invention may he used to detect a target substance in any test sample. Thus this invention also provides a method for detecting the presence or absence of a substance in a test sample. Typically, the test sample will be a fluid Probes are typically used as substantially purified proteins. Alternatively, living cells, for example bacterial cells, that express a probe (or probes) may be used.

Typically, a method for detecting the presence or absence of a substance comprises: determining the amount of FRET from a probe or cell harbouring a probe, contacting the probe or cell with a test sample; determining any change in. FRET from the probe or cell; thereby to determine the presence or absence of the target substance in the sample. As well as giving a qualitative indication of the presence or absence of a target substance, the method of the invention may also provide a quantitative measurement of the amount of the substance present in the test sample.

Probes of the invention may be used singly or in combination. Thus, two or more, for example, three, five, ten or more, may be used simultaneously in a method of the invention for detecting the presence or absence of a test sample. Typically, if more than one probe of the invention is used simultaneously, the donor and acceptor polypeptides of each probe will be different. When more than one probe is used, preferably any probe used will not interfere with the ability of another probe to undergo FRET. FRET from each probe can be measured sequentially or simultaneously, using appropriate detection apparatus. The use of more than one probe in a method of the invention for detecting the presence or absence of a substance will allow the presence or absence of more than one substance in a test sample to be determined.

When using a substantially purified probe, any suitable technique may be used to detect the presence or absence of a test sample. One of the following two approaches is typically used:
(1) Equilibrium approach - a probe which has an affinity comparable to the typical concentration of the target substance is used, but at a comparatively low absolute amount. In this way, only a small proportion of the population of target substance molecules binds the probe molecules and thus the concentration of the target substance is not markedly affected.
   Calibration of the probe may be carried out using media comprising known amounts of the target substance. Suitable controls may be used, for example media in which the target substance is not present may be used. Also, competition experiments between the unknown and known amounts of the target substance may be carried out to test for interference by other compounds present in the test sample. Additionally, the probe may be washed out after a test and recalibrated to test for irreversible modification of the probe.
(2) Saturation (affinity) approach - the probe has a very high affinity for the target substance in comparison to the concentration of the target substance and is present in amounts such that the test sample is substantially depleted in respect of the target substance. This approach may be used in static systems, whereby the probe is placed in contact with a known volume of the sample or in a flow-through system whereby a solution of the test sample and/or controls are passed over the immobilized probe. Similar controls may be used to those described for (1) above. In addition, when a sensor comprising immobilized probe is used, the profile of binding along the length of the sensor can also be monitored and analysed to calculate the binding affinity of the target and probe.

When a probe is used to detect the presence or absence of a substance and that probe is harboured by a cell, appropriate assay methods may be more complex. Calibration of the probe is preferred where known concentrations of the target have somehow been introduced into the cell. For ions, this may be carried out through the use of ionophores. For organic molecules, a non-specific permeabilisation agent, for example streptolysin, may be used in a medium containing known amounts of the target substance. Alternatively, the calibration is based on the response of a probe determined in a medium designed to mimic the environment of the probe within the cell.

Probes of the invention may be incorporated into a sensor. Preferably, such a sensor is small and portable. Thus the present invention also provides a sensor comprising a probe of the invention, a light source which is capable of exciting the probe and a detector which is capable of measuring the amount of FRET. A typical sensor is illustrated in Figure 3. The sensor is generally based on silicon chips with five modules per probe: (i) a blue-light emitting diode or small blue laser, or an LED or small laser of a different wavelength if the donor fluorescent polypeptide responds to a different wavelength of light; (ii) a pad for immobilising the probe, accessible to (iii) a sample delivery/flow-through system; (iv) a first silicon detector; and (v) a second silicon detector, wherein the two silicon detectors have different spectral sensitivities to measure the fluorescence from the two fluorescent polypeptides of the probe.

A typical low-cost detector comprises two silicon devices equipped with interference filters or coloured-glass filters with an appropriate peak transmission and bandwidth. Such detectors can be used singly or optionally can be arranged in arrays. Cooling to -20°C may be required in some situations to achieve a good signal-to-noise ratio. A more complex system comprises a diode-array detector preceded by a prism or diffraction grating so that a complete emission spectrum can be collected, rather than just two emission wavelengths. Complete emission spectra may contain more information about whether the change in signal is entirely due to changes in FRET.

A sensor may also be in the form of a dual-multiplier system with light separated into two channels using a dichroic mirror and each channel equipped with an appropriate filter. Alternatively, the two photomultipliers with appropriate filters could be placed adjacent to the sample chamber as indicated for the silicon detector system described above.

For remote applications, where complex electronics are not possible or undesirable, sensors based on film or phosphor imager plates would be suitable.

To minimize the amount of direct illumination received by the detectors, the detectors should each generally not be oriented at 180°C or near 0°C to the light source. Typically, for a probe immobilised on an opaque light substrate the detectors should be at an angle of about 45°C to the light source. The sensor may comprise a flow-through cell with an array, typically parallel, of different probes so that the presence of numerous target substances can be determined simultaneously. Additionally a flow-through cell may comprise a series/array of probes specific for the same substance may be used which differ in affinity for the target substance because, for example, the probes have linkers with different flexibilities.

Probes of the invention may be used to detect the presence of a substance, for example a metabolite, hormone, drug, toxin or pollutant in an extract, for example a fluid sample derived from any organism, including an animal or human, plant, fungus or microbe.

For example, a probe of the invention may be used to detect sugars, oligosaccharides or non-carbohydrate mimetics. For such use, the target binding site of the probe comprises a recombinant monomeric plant lectin of the desired oligosaccharide binding specificity. The mimic peptide is endogenously biotinylated. The probe is activated by the attachment of a small biotinylated glycoprotein, to generate an interaction between the lectin and the cognate oligosaccharide recognition element with the consequent appearance of a FRET signal. Sugar, oligosaccharide or non-carbohydrate mimetics may then be detected by their ability to reduce this FRET signal.

A probe of the invention may also be used to determine the presence or absence or steroid hormones. Such an application makes use of the change in binding affinity of a sythetic peptide probe to a steroid hormone receptor, for example the estrogen receptor (ER) upon binding of a specific steroid hormone (Paige *et al*., 1999, Proc. Natl. Acad. Sci. USA **96**, 3999-4004). The target binding site of a suitable probe may comprise sequence encoded by a cDNA corresponding to the estrogen receptor. The mimic peptide comprises one of a least three sequences:
(i) SSNHQSSRLIELLSR (this sequence shows no binding to ER except in the presence of estradiol).
(ii) SAPRATISHYLMGG (this sequence binds ER in the absence of steroids, but is released by estradiol or tamoxifen); or
(iii) SSPGSREWFKDMLSR (this sequence shows no binding to ER except in the presence of tamoxifen.

For sequences (i) and (ii), the probe operates in the opposite manner to that generally described above. That is, the target binding site peptide and mimic do not freely bind each other in the absence of the target substance. Rather only in the presence of the target substance do the target binding site peptide and mimic peptide bind. Thus, in such cases the presence of the target substance will lead to a reduction in the separation of the fluorophors and therefore to an *increase* in FRET. In the description of probes above, typically the presence of the target substance is indicated by a *decrease* in FRET.

In the case of an animal or human, the sample could be, for example, blood, saliva, tears, cerebro-spinal fluid or semen. A probe may be used to determine the presence or absence of a particular substance in an animal or human sample. The presence of a particular substance in a substance may be indicative of a disease state. Alternatively, the absence of a particular substance may be indicative of a disease/clinical condition. Thus the invention provides a probe for use in a method of diagnosis practised on the human or animal body.

The invention also provides a method of diagnosis comprising determining the amount of FRET from a probe and then contacting an animal or human sample with a probe of the invention. Any change in FRET is determined and thereby the presence or absence of a particular target substance is determined. The disease state, healthy or otherwise, of the animal or human may thus be determined. The method is typically carried out *ex vivo*, ie. on a sample withdrawn from the subject.

Other applications in animals or humans include drug and alcohol testing and testing for exposure to toxins or pollutants.

A probe of the invention may also be used to detect air-borne substances, for example atmospheric pollutants, if these substances are soluble. Thus, a probe of the invention can be provided in an aqueous medium which is exposed to the surrounding atmosphere. Any substances in the surrounding air which are soluble will dissolve in the probe containing medium and can be detected by a suitable probe or probes in the medium.

Probes of the invention may also be used to detect specific substances in plant, fungal or microbial, for example bacterial, extracts. Plant extracts, for example exudates, may be useful in determining the presence of plant pathogenic viruses or bacteria in a plant. Additionally, probes of the invention may be used to determine the presence and amount of trace elements or pollutants in plant extracts. Thus results or such assays may provide indirect measurements of soil quality and in some cases be indicative of particular types of soil pollution.

A further application of probes of the invention is to use them to detect proteins expressed in transgenic plants, or transgenic animals, fungi or microbes. When transgenic organisms are produced, often large numbers of so-called primary transformants have to be screened for expression of the transgene. Typically, time-consuming RNA and protein blotting techniques are used. Probes of the invention could be used to assay crude extracts in a more quantitative fashion that RNA and protein blotting and also more quickly than those techniques.

Probes may be used to detect for example contaminants or pollutants in for example, water supplies, soil or factory effluents. Probes may be used in quality control situations to detect substances, for example contaminants, in foodstuffs and medicaments.

This invention also provides multicellular organisms or parts thereof comprising a probe, polynucleotide, vector or cell of the invention. Typically such organisms will comprise a polynucleotide of the invention, such that the probe for which that polynucleotide codes is expressed in that organism or part thereof. In other words, the organisms or parts thereof may be transgenic for a polynucleotide of the invention. An organism or part thereof may comprise more than one polynucleotide, vector, cell or probe of the invention.

The expression of a probe may be constitutive or tissue specific and may persist for the whole of the organisms life-cycle or may be expressed at a particular developmental stage of the life-cycle. Different probes may be expressed at different times during the life-cycle of the organism. Thus organisms may be produced, wherein the probe is expressed under the control of a constitutive promoter or under the control of a promoter which directs spatially or temporally restricted expression. Suitable promoters are well known to those skilled in the art.

Any multicellular organism may comprise a probe, nucleotide, vector or cell or the invention, for example, fungi, plants and non-human animals. Suitable plants may be monocotyledonous or dicotyledonous. Preferred monocots are graminaceous plants such as wheat, maize, rice, oats, barley and rye, sorghum, triticale and sugar cane. Preferred dicotyledonous crop plants include tomato, potato, sugarbeet and other beet crops; cruciferous crops, including oilseed rape; linseed; tobacco; sunflower; fibre crops such as cotton; and leguminous crops such as peas, beans, especially soybean, and alfalfa. Suitable animals include insects, for example the dipteran *Drosophila melanogaster* and mammals, for example mice, sheep, pigs or cows.

Multicellular organisms comprising probes, polynucleotides, vectors or cells of the invention may be generated according to techniques well-known to those skilled in the art. Generally, a polynucleotide of the invention is incorporated into a vector and that vector is used to transform or transfect a cell of the organism. That cell is then used to regenerate a multicellular organism, which will generally be able to replicate. Thus, the invention also provides a method of producing a transgenic organism which comprises transforming or transfecting a single cell of that organism with a polynucleotide of the invention and allowing that cell to develop into a multicellular organism.

Use of probes of the invention in living cells falls into two main classes: (i) use in isolated cells in culture; and (ii) use in intact multicellular organisms.

Isolated cells in culture may be microbial, for example bacterial, fungal, plant or animal, for example mammalian cells, which comprise a probe or probes of the invention. The probe or probes could be to any substance or substances including: metabolites, for example glucose, sucrose and NADPH; signalling molecules, for example Ca2', H', Ins(1,4,5)P3, cAMP, cGMO, testosterone, xenobiotics, for example toxins, drugs, herbicides, pesticides or fungicides; peptides such as calmodulin or kinases, post-translational modification sites, for example phosphorylation, glycosylation or farnesylation sites.

Cells containing probes may be grown in suitable media in, for example, multi-well plates or microscope chambers. Changes in FRET may be recorded using, for example, a fluorimeter, fluorescent plate reader, camera imaging system, confocal microscope or multi-photon microscope.

Assays may be for the indirect effects of a drug on the metabolism or physiology of a cell, rather than as a direct probe for the presence of a drug. Such systems can form the basis of high-throughput physiological screening systems. For example, in the case of a therapeutic drug which as well as having a therapeutic effect has unwanted side-effects, substances could be screened for their ability to reduce the side-effect. A probe is used which is specific for a physiological indication of the side-effect, for example, increased accumulation of a particular metabolite. Collections of substances, for example combinatorial libraries, could be screened for in high-throughput assays for substances which prevent increase of the metabolite and thus have the potential to ameliorate side-effects of the drug.

In multicellular systems the approach is similar to that outlined above for single cells, however, typically only the surface cell layers of a multicellular organism are accessible to non-invasive fluorescence techniques. Global fluorescence measurements can be made using photometry, fluorimetry, camera, confocal or multi-photon techniques. Tissue, cell or organelle specificity can be achieved using tissue-specific, developmental-specific and/or targeted probes, ie. probes that are expressed under the control of tissue- or developmental- specific probes or probes that comprise a targetting peptide.

For example, to determine changes in the plant hormone abscisic acid in the stomatal guard cells of a leaf, a probe directed to abscisic acid is expressed only in those cells. Changes in FRET of the probe are monitored using a non-imaging system. Alternatively, the probe is expressed constitutively throughout the plant, in which case measurements are made only from the guard cells using an imaging technique.

Probes of the invention may also be used to investigate binding between two substances, which two substances would typically bind to each other. Thus, the invention also provides a method for identifying an inhibitor of binding between two substances, which two substances would bind to each other in the absence of an inhibitor.

The types of binding interaction that may be investigated may be, for example, peptide-peptide interactions, peptide-carbohydrate, peptide-nucleic acid or peptide-ligand interactions. If a target binding site peptide and mimic peptide pair can be can identified, the interaction of which mimics the binding interaction of the two substances of interest, the interaction between those two substances can be investigated.

Typically, such methods will be used to investigate interactions which are of significance in human or animal disease states. For example, host recognition by a pathogen is often a critical step in infection. Probes of the invention may be used to investigate that pathogen-host recognition interaction. For example, some pathogens recognise carbohydrate species on the surface of host cells. An appropriate probe may be designed which can be used to identify inhibitors of the binding interaction between a pathogen and a carbohydrate molecule on the surface of a host cell. An inhibitor so identified may be used to disrupt the recognition interaction between the host and pathogen and therefore may be used to prevent infection of the host by the pathogen.

Thus, inhibitors identified by a probe of the invention may be used in a method of treatment of the human or animal body by therapy.

Probes of the invention may be designed for use in identifying inhibitors of estrogen stimulated transcription. Such a probe comprises the estrogen receptor (ER) as the target binding site peptide and is biotinylated at the mimic peptide. Therefore a biotinylated oligonucleotide bearing the estrogen receptor response element (ERE) can be attached to the mimic peptide. The ER will bind to the ERE and give a FRET signal unless an inhibitor of the DNA-protein interaction is present, in which case the FRET signal will be lost. Therefore such a probe could be used to screen for inhibitors of the growth of estrogen-sensitive breast tumors. Such a screen could be used to identify anti-tumor agents that act at a site distinct from that targeted by the synthetic estrogen, tamoxifen.

Probes of the invention can also be use to identify protease inhibitors. The target binding site of a suitable probe comprises a recombinant protease and the mimic peptide comprises a known peptide inhibitor. Thus FRET is detected in the resting state as the inhibitor binds in the protease active site. The probe can thus be used to screen for active binding site inhibitors of the protease.

Probes of the invention may further be used to identify intracellular G protein signal inhibitors. Thus, probes can be used to identify novel classes of signal transduction inhibitor. In a suitable probe, the target site binding peptide comprises the cytoplasmic loop of a selected seven transmembrane receptor and the other end comprises the C terminal part of an alpha subunit of a heterotrimeric G protein complex. Since the C terminal region of the alpha subunit contains the receptor binding site and is functional in isolation, the probe displays FRET in the resting state. An inhibitor of this interaction would reduce the FRET signal.

Typically, a method for identifying an inhibitor of a binding interaction between two substances may be carried out by determining the amount of FRET from a suitable probe in the absence of a test substance; contacting the probe with a test sample; and determining the FRET from the probe thereby to determine whether the test sample can inhibit the binding interaction between the two substances of interest. Inhibition of the binding interaction will typically be indicated as a reduction of FRET of the probe.

Suitable control experiments can be carried out. For example, a candidate inhibitor can be tested with other probes-of the invention, to determine that it specifically inhibits the interaction under investigation and is not simply a general, non-specific inhibitor of many binding interactions.

Any suitable format can be used for carrying out a method for identifying an inhibitor of a binding interaction. However, the screening method is preferably carried out in a single medium, most preferably in a single well of a plastics microtitre plate. Thus the method can be adapted for use in high though-put screening techniques.

Suitable test substances for inhibitors of binding interactions include combinatorial libraries, defined chemical entities, peptides and peptide mimetics, oligonucleotides and natural product libraries. The test substances may be used in an initial screen of, for example, ten substances per reaction, and the substances of batches which show inhibition tested individually. Furthermore, antibody products (for example, monoclonal and polyclonal antibodies, single chain antibodies, chimaeric antibodies and CDR-grafted antibodies) may be used.

## Claims

1. A probe comprising:
(i) a target binding site peptide which is attached to a first fluorescent polypeptide;
(ii) a mimic peptide which is capable of binding to the target binding site peptide and is attached to a second fluorescent polypeptide; and
(iii) a linker which connects the two fluorescent polypeptides and which allows the distance between said fluorescent polypeptides to vary, said fluorescent polypeptides being such as to display fluorescence resonance energy transfer (FRET) between them.

2. A probe according to claim 1, wherein the linker is a peptide or polypeptide.

3. A probe according to claim 2, which is a single polypeptide.

4. A probe according to any one of the preceding claims, wherein the first fluorescent polypeptide is a green fluorescent protein (GFP).

5. A probe according to any one of claims 1 to 3, wherein the second fluorescent polypeptide is a GFP.

6. A probe according to any one of the preceding claims, wherein the mimic peptide comprises a sequence capable of biotinylation.

7. A probe according to claim 6 which is biotinylated.

8. A polynucleotide which encodes a probe according to any one of claims 3 to 7.

9. A polynucleotide according to claim 8 which is a DNA sequence.

10. A vector incorporating a polynucleotide according to claim 8 or 9.

11. A vector according to claim 10, which is an expression vector.

12. A cell harbouring a probe according to any one of claims 1 to 7, a polynucleotide according to claim 8 or 9 or a vector according to claim 10 or 11.

13. A fungus, plant or non-human animal comprising a probe according to any one of claims 1 to 7, a polynucleotide according to claim 8 or 9, a vector according to claim 10 or 11 or a cell according to claim 12.

14. A sensor comprising:
(i) a probe according to any one of claims 1 to 7;
(ii) a light source which is capable of exciting the probe;
(iii) a detector which is capable of measuring the amount of FRET from the probe.

15. A sensor according to claim 14, wherein there are two detectors, one of which is sensitive to the first fluorescent polypeptide of the probe and the other of which is sensitive to the second fluorescent polypeptide of the probe.

16. A sensor according to claims 14 or 15 which comprises more than one probe.

17. A method for detecting the presence or absence of a target substance in a test sample comprising:
(i) providing a probe according to any one of claims 1 to 7, a cell according to claim 12 or a sensor according to any one of claims 14 to 16, wherein the target binding site peptide of the probe, cell or sensor is capable of binding to the target substance;
(ii) determining the amount or FRET of the probe, cell or sensor;
(iii) contacting the probe, cell or sensor with the test sample; and
(iv) determining any change in FRET thereby to determine whether the test sample comprises the target substance.

18. Use of a probe according to any one of claims 1 to 7, a cell according to claim 12 or a sensor according to any one of claims 14 to 16, wherein the target binding site peptide of the probe, cell or sensor is capable of binding to a target substance, in the detection of the presence or absence of that target substance in a test sample.

19. A method for identifying an inhibitor of binding between two substances, which two substances would bind to each other in the absence of an inhibitor, comprising:
(i) providing a probe according to any one or claims 1 to 7 or a cell according to claim 12, wherein the binding of the target site peptide to the mimic peptide mimics the binding of the two substances to each other;
(ii) determining the amount of FRET of the probe or cell;
(iii) contacting the probe or cell with a test substance; and
(iv) determining any change in FRET thereby to determine whether the test substance inhibits binding between the two substances.

20. Use of a probe according to any one of claims 1 to 7 or a cell according to claim 12, wherein the binding of the target site peptide to the mimic peptide mimics the binding of two substances to each other, in the identification of an inhibitor of binding between those two substances.

## Patentansprüche

1. Sonde, umfassend:
(i) ein Ziel-Bindungsstellen-Peptid, welches an ein erstes Fluoreszenz-Polypeptid gebunden ist;
(ii) mimisches Peptid bzw. Imitator-Peptid, welches in der Lage ist, an ein Ziel-Bindungsstellen-Peptid zu binden und an ein zweites Fluoreszenz-Polypeptid gebunden ist; und
(iii) einen Linker, welcher die zwei Fluoreszenz-Polypeptide verbindet und welcher es ermöglicht, den Abstand zwischen den Fluoreszenz-Polypeptiden zu variieren, wobei die Fluoreszenz-Polypeptide dergestalt sind, das zwischen ihnen ein Fluoreszenz-Resonanz-Energietransfer (FRET) gezeigt wird.

2. Sonde gemäß Anspruch 1, wobei der Linker ein Peptid oder Polypeptid ist.

3. Sonde gemäß Anspruch 2, welche ein einzelnes Polypeptid ist.

4. Sonde gemäß mindestens einem der vorhergehenden Ansprüche, wobei das erste Fluoreszenz-Polypeptid ein Grün-Fluoreszenz-Protein (GFP) ist.

5. Sonde gemäß mindestens einem der Ansprüche 1 bis 3, wobei das zweite Fluoreszenz-Polypeptid GFP ist.

6. Sonde gemäß mindestens einem der vorhergehenden Ansprüche, wobei das mimische Peptid eine Sequenz umfasst, die zur Biotinylierung in der Lage ist.

7. Sonde gemäß Anspruch 6, welche biotinyliert ist.

8. Polynukleotid, welche eine Sonde gemäß mindestens einem der Ansprüche 3 bis 7 umfasst.

9. Polynukleotid gemäß Anspruch 8, welches eine DNA-Sequenz ist.

10. Vektor, in den ein Polynukleotid gemäß Anspruch 8 oder 9 eingebaut ist.

11. Vektor gemäß Anspruch 10, welcher ein Expressionsvektor ist.

12. Zelle, die eine Sonde gemäß mindestens einem der Ansprüche 1 bis 7, ein Polynukleotid gemäß Anspruch 8 oder 9 oder einen Vektor gemäß Anspruch 10 oder 11 beherbergt.

13. Pilz, Pflanze oder nicht-humanes Tier, umfassend eine Sonde gemäß mindestens einem der Ansprüche 1 bis 7, ein Polynukleotid gemäß Anspruch 8 oder 9, einen Vektor gemäß Anspruch 10 oder 11 oder eine Zelle gemäß Anspruch 12.

14. Sensor, umfassend
(i) eine Sonde gemäß mindestens einem der Ansprüche 1 bis 7;
(ii) eine Lichtquelle, welche zur Anregung der Sonde in der Lage ist;
(iii) einen Detektor, welcher zum Messen der Menge an FRET von der Sonde in der Lage ist.

15. Sensor gemäß Anspruch 14, worin es zwei Detektoren gibt, wobei einer davon gegenüber dem ersten Fluoreszenz-Polypeptid empfindlich ist und der andere gegenüber dem zweiten Fluoreszenz-Polypeptid der Sonde empfindlich ist.

16. Sensor gemäß Anspruch 14 oder 15, welcher mehr als eine Sonde umfasst.

17. Verfahren zum Nachweis der Anwesenheit oder Abwesenheit einer Zielsubstanz in einer Testprobe, umfassend:
(i) Vorsehen einer Sonde gemäß mindestens einem der Ansprüche 1 bis 7, einer Zelle gemäß Anspruch 12 oder eines Sensors gemäß mindestens einem der Ansprüche 14 bis 16, wobei das Ziel-Bindungsstellen-Peptid der Sonde, der Zelle oder des Sensors zum Binden der Zielsubstanz in der Lage ist;
(ii) Bestimmen der Menge an FRET der Sonde, der Zelle oder des Sensors;
(iii) Kontaktieren der Sonde, der Zelle oder des Sensors mit der Testprobe; und
(iv)Bestimmen jedweder Änderung im FRET, um hierdurch zu bestimmen, ob die Testprobe die Zielsubstanz umfasst.

18. Verwendung einer Sonde gemäß mindestens einem der Ansprüche 1 bis 7, einer Zelle gemäß Anspruch 12 oder eines Sensors gemäß mindestens einem der Ansprüche 14 bis 16, wobei das Ziel-Bindungsstellen-Peptid der Sonde, der Zelle oder des Sensors zum Binden an eine Zielsubstanz in der Lage ist, bei dem Nachweis der Anwesenheit oder Abwesenheit der Zielsubstanz in einer Testprobe.

19. Verfahren zum Identifizieren eines Inhibitors der Bindung zwischen zwei Substanzen, wobei die zwei Substanzen in Abwesenheit eines Inhibitors binden würden, umfassend:
(i) Vorsehen einer Sonde gemäß mindestens einem der Ansprüche 1 bis 7 oder einer Zelle gemäß Anspruch 12, wobei das Binden des Zielstellen-Peptids an das mimische Peptid das aneinander Binden der zwei Substanzen nachahmt;
(ii) Bestimmen der Menge an FRET der Sonde oder der Zelle;
(iii) Kontaktieren der Sonde oder der Zelle mit einer Testsubstanz; und
(iv)Bestimmen jedweder Änderung im FRET, um **dadurch** zu bestimmen, ob die Testsubstanz die Bindung zwischen den zwei Substanzen inhibiert.

20. Verwendung einer Sonde gemäß mindestens einem der Ansprüche 1 bis 7 oder einer Zelle gemäß Anspruch 12, wobei das Binden des Zielstellen-Peptids an das mimische Peptid das aneinander Binden von zwei Substanzen nachahmt, beim Nachweis eines Inhibitors der Bindung zwischen diesen zwei Substanzen.

## Revendications

1. Sonde-comprenant :
(i) un peptide de site de liaison cible qui est attaché à un premier peptide fluorescent ;
(ii) un peptide mimétique qui est capable de se lier au peptide de site de liaison cible et qui est attaché à un second polypeptide fluorescent ; et
(iii) un bras espaceur qui relie les deux polypeptides fluorescents et qui permet de faire varier la distance entre lesdits peptides fluorescents, lesdits peptides fluorescents étant tels qu'ils présentent entre eux un transfert d'énergie de fluorescence par résonance (FRET).

2. Sonde selon la revendication 1, dans laquelle le bras espaceur est un peptide ou un polypeptide.

3. Sonde selon la revendication 2, qui est un polypeptide unique.

4. Sonde selon l'une quelconque des revendications précédentes, dans laquelle le premier polypeptide fluorescent est une protéine fluorescente verte (GFP).

5. Sonde selon l'une quelconque des revendications 1 à 3, dans laquelle le second polypeptide fluorescent est une GFP.

6. Sonde selon l'une quelconque des revendications précédentes, dans laquelle le peptide mimétique comprend une séquence pouvant être biotinylée.

7. Sonde la revendication 6 qui est biotinylée.

8. Polynucléotide qui code une sonde selon l'une quelconque des revendications 3 à 7.

9. Polynucléotide selon la revendication 8 qui est une séquence d'ADN.

10. Vecteur incorporant un polynucléotide selon la revendication 8 ou 9.

11. Vecteur selon la revendication 10, qui est un vecteur d'expression.

12. Cellule portant une sonde selon l'une quelconque des revendications 1 à 7, un polynucléotide selon la revendication 8 ou 9 ou un vecteur selon la revendication 10 ou 11.

13. Champignon, plante ou animal non humain comprenant une sonde selon l'une quelconque des revendications 1 à 7, un polynucléotide selon la revendication 8 ou 9, un vecteur selon la revendication 10 ou 11 ou une cellule selon la revendication 12.

14. Capteur comprenant :
(i) une sonde selon l'une quelconque des revendications 1 à 7 ;
(ii) une source lumineuse qui est capable d'exciter la sonde ;
(iii) un détecteur qui est capable de mesurer la quantité de FRET provenant de la sonde.

15. Capteur selon la revendication 14, dans lequel il y a deux dispositifs de détection, un qui est sensible au premier polypeptide fluorescent de la sonde et l'autre qui est sensible au second polypeptide fluorescent de la sonde.

16. Capteur selon la revendication 14 ou 15 qui comprend plus d'une sonde.

17. Procédé pour détecter la présence ou l'absence d'une substance cible dans un échantillon à tester, comprenant :
(i) le fait de fournir une sonde selon l'une quelconque des revendications 1 à 7, une cellule selon la revendication 12 ou un capteur selon l'une quelconque des revendications 14 à 16, le peptide de site de liaison cible de la sonde, de la cellule ou du détecteur étant capable de se lier à la substance cible ;
(ii) le fait de déterminer la quantité de FRET de la sonde, de la cellule ou du capteur ;
(iii) le fait de mettre en contact la sonde, la cellule ou le capteur avec l'échantillon à tester ; et
(iv) le fait de déterminer un changement quelconque de FRET pour déterminer si l'échantillon à tester comprend la substance cible.

18. Utilisation d'une sonde selon l'une quelconque des revendications 1 à 7, d'une cellule selon la revendication 12 ou d'un capteur selon l'une quelconque des revendications 14 à 16, le peptide de site de liaison cible de la sonde, de la cellule ou du détecteur étant capable de se lier à une substance cible, dans la détection de la présence ou de l'absence de cette substance cible dans un échantillon à tester.

19. Procédé pour identifier un inhibiteur de liaison entre deux substances, lesquelles deux substances se lieraient l'une à l'autre en l'absence d'un inhibiteur, comprenant :
(i) le fait de fournir une sonde selon l'une quelconque des revendications 1 à 7 ou une cellule selon la revendication 12, la liaison du peptide de site de liaison cible au peptide mimétique imitant la liaison des deux substances l'une à l'autre ;
(ii) le fait de déterminer la quantité de FRET de la sonde ou de la cellule ;
(iii) le fait de mettre la sonde ou la cellule en contact avec une substance à tester ; et
(iv) le fait de déterminer un changement quelconque de FRET pour déterminer si la substance testée inhibe la liaison entre les deux substances.

20. Utilisation d'une sonde selon l'une quelconque des revendications 1 à 7 ou d'une cellule selon la revendication 12, dans laquelle la liaison du peptide de site cible au peptide mimétique imite la liaison de deux substances l'une à l'autre, dans l'identification d'un inhibiteur de liaison entre ces deux substances.
